# EUROPEAN PATENT APPLICATION

(11) **EP 3 298 969 A1**
(43) Date of publication of application: **28.03.2018**
(21) Application number: 16306228.4
(22) Date of filing: 26.09.2016
(51) Int. Cl.: A61B 17/04, A61B 17/06, A61B 17/068

(54) **SURGICAL SUTURE DEVICE**

(71) Applicant: CHU de NICE, 06200 Nice (FR)
(72) Inventor: SEJOR, Eric, 06000 NICE (FR)
(74) Representative: Osha Liang

(57) **Abstract**

A surgical suture device (100) for suturing body tissues comprising two hollow needles (120), each having a longitudinal lumen (121), a suture thread (20) with two opposite ends (22) which are movable, respectively, inside and along the two longitudinal lumens (121), a projection mechanism (130) for projecting the suture thread ends (22) out of the needles (120), wherein each suture thread end (22) forms an anchoring head (10) for engagement with body tissues (1, 2) or a lock-in holder.

## Description

### TECHNICAL FIELD

The present disclosure relates generally to a medical device. In particular, it relates to a surgical suture device for suturing body tissues.

### BACKGROUND

It is commonly known to close a wound or a tissue opening with staples or with a suture thread and a hand-operated needle. However, in open surgery, staples are generally not used for closing deep tissue opening because of the infectious risks. "Deep tissues", e.g. muscular and facial tissues, are body tissues which are located under the skin and the subcutaneous fat (forming the superficial tissues). Thus, the closure of deep tissues is generally hand-made by the practitioner with a hand-operated needle and a suture thread.

As an alternative to hand-operated needles, instruments with mechanisms for driving a needle through body tissues and suturing them have been proposed. An example of such a mechanical instrument is described, for instance, in patent document US 2009/0264905 A1. The mechanical instrument comprises a first hollow needle for insertion of a suture thread and a second hollow needle for gripping the suture thread. A suture thread is inserted into the first needle. A stylet is slidably inserted into the inside of the second needle. The stylet is provided with an annular member at the distal end thereof to be projected toward the first needle. When the annular member is projected from the distal end of the second needle, the annular member extends toward the first needle such that said first needle penetrates through the inside of the annular member and the suture thread passes through the inside of the annular member.

However, there still exists a need for new alternatives to hand-operated needles, in particular for suturing deep body tissues in open surgery.

### GENERAL PRESENTATION

According to one aspect of the present disclosure, a surgical suture device comprising two hollow needles, each having a longitudinal lumen, is provided. The device also comprises a suture thread with two opposite ends which are movable, respectively, inside and along the two longitudinal lumens. The device further comprises a projection mechanism for projecting the suture thread ends out of the needles, each suture thread end forming an anchoring head for engagement with body tissues or a lock-in holder.

For closing a tissue opening with such a surgical suture device, the two hollow needles may be moved, respectively, through two portions of tissues on each side of the opening. In other terms, each portion of tissues is punctured by a needle so that the distal end of the needle extends beyond the tissue portion. Then, the suture thread ends are moved inside the needles toward their distal end and projected out of the needle (i.e. out of the longitudinal lumen) by the projection mechanism. Finally, each anchoring head formed by the suture thread end anchors itself in the tissue portion. As an alternative, the anchoring head anchors itself in a lock-in holder, thereby forming a block which, in turn, gets caught on the tissue portion. The two opposite anchoring heads of the suture thread are thus caught on a first side of the tissue layer to be sutured, while being bonded by a link formed by the central part of the suture thread. The link begins from one of the anchoring heads, goes through the thickness of the tissue layer, goes along the second side of the tissue layer across the opening line, goes through the thickness of the tissue layer again, and ends at the other anchoring head. As a result, both tissue portions are bonded together and the tissue opening is held closed. Both tissue portions are thus sutured together while limiting the practitioner's contribution, as compared to conventional hand-operated needles. Also, because of the anchoring heads, there is no need for the practitioner to knot the suture thread ends.

Such a device makes it possible to perform surgical sutures quite easily and rapidly, in a reproducible and effective manner. Compared to other devices or manual suturing with hand-operated needles, it is also well adapted for suturing deep body tissues in open surgery. In particular, it allows bringing together two deep tissue portions and maintaining them close together without having to hold a pull or strain on the suture thread (which can sometimes lead to rupture of the thread) as is typically done in manual suturing of deep tissues. Furthermore, the device allows distributing the forces exerted by the thread on the tissue in an homogeneous way. This ensures more effective healing and, as a result, a lower risk of suture failure, of evisceration, and of eventration.

In the present disclosure, the terms "body tissues" encompasses any kind of body tissues, being animal or human tissues. Also, the terms "distal" and "proximal" are used from the point of view of the practitioner holding the device. In other words, the distal end of the needle is the pointed tip of the needle.

The anchoring head formed by the suture thread may have different shapes, including a pyramidal, conical or frustroconical shape, an arrow shape, an umbrella shape, a Christmas tree shape, etc. or any shape adapted for engagement with body tissues or a lock-in holder. In certain embodiments, the suture thread end has a T-shape, the anchoring head being formed by the head, or upper bar, of the T.

In certain embodiments, the surgical suture device comprises a lock-in holder cooperating with the anchoring head.

In certain embodiments, the anchoring heads of the suture thread are connected with each other by a first link. As already mentioned, the first link corresponds to the central part of the suture thread extending between the two opposite anchoring heads. The first link may have different structures, shapes and properties. For instance, it may comprise one or more filaments, it may be bioabsorbable or not, etc.

In certain embodiments, the surgical suture device comprises a lock-in unit with two lock-in holders connected with each other by a second link. Similar to the first link, the second link may have different structures, shapes and properties.

In certain embodiments, each of the needles comprises a longitudinal slot extending along the longitudinal lumen and communicating therewith, and the first link passes through and is movable along the longitudinal slot. The first link thus extends from one needle to the other needle, while being movable in the longitudinal direction of the needles.

In certain embodiments, each of the needles has a distal end portion, or tip portion, with a side opening for discharging the suture thread end. The longitudinal lumen ends at the side opening. Before the side opening, the internal wall of the longitudinal lumen may be curved to help discharge the suture thread end through the side opening.

In certain embodiments, each of the needles has a tip and the device comprises an anvil distally spaced from the needle tips. The anvil may hold the lock-in holders. The anvil allows the body tissues, which do not need to be sutured, to be protected from the needles and from the suture thread ends projected out of needles. The suture thread ends may come into contact with the anvil when they are projected, which may help to change the orientation of the suture thread ends.

Other features and advantages of the invention will become apparent from the following detailed description, taken in conjunction with the accompanying drawings, which illustrate, by way of example, the principles of the invention.

### BRIEF DESCRIPTION OF THE DRAWINGS

In the drawings, like reference signs generally refer to the same or like parts throughout the different views. The drawings are not necessarily to scale, emphasis instead being placed upon illustrating the principles of the invention.
FIG 1 illustrates an example of a surgical suture device when the device punctures two tissue portions to be sutured.
FIG 2 is a detailed view of the device of FIG 1 when the suture thread ends are projected out of the needles.
FIG 3 is a detailed view of the suture thread ends engaged with the two tissue portions, the needles being removed from the body tissues.
FIGS 4A to 4C illustrate examples of suture threads.
FIG 5 illustrates an example of a lock-in holder.
FIG 6 illustrates two tissue portions sutured by a suture thread and the lock-in holder of FIG 5.
FIG 7 is a detail view of a distal end portion of a needle.

### DETAILED DESCRIPTION

The accompanying drawings showing an example of a surgical suture device are referred to in the following detailed description. It is intended that this example be considered as illustrative only, the invention not being limited to this example.

To avoid unnecessary details for practicing the invention, the description may omit certain information already known to those skilled in the art.

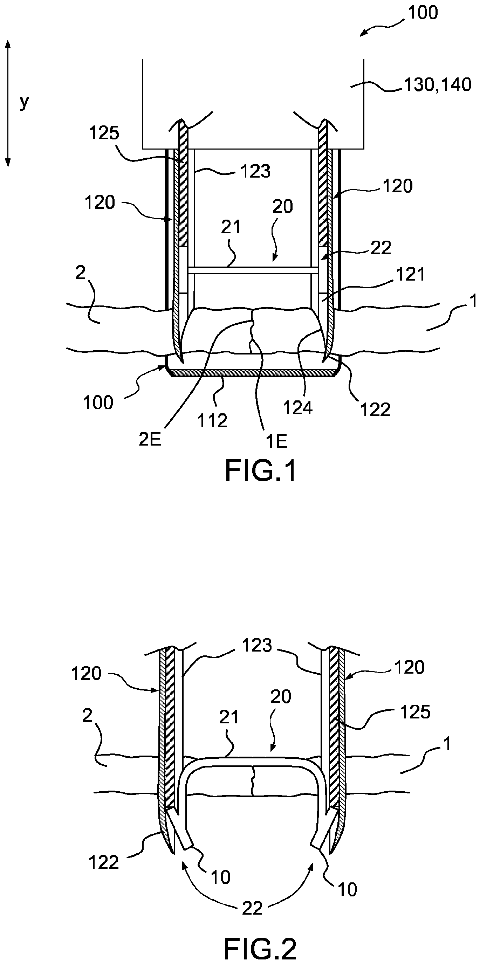

FIG 1 illustrates a surgical suture device 100 used for suturing two portions of tissues 1 and 2 together. The two tissue portions 1, 2 are located on each side of an opening and their edges 1E, 2E are brought in contact with each other to close the opening.

The surgical suture device 100 comprises two hollow needles 120, each needle comprising a longitudinal lumen 121. The hollow needles 120 may be parallel with each other. The longitudinal lumen 121 opens out more or less close to the tip 122 of the needle 120, thus forming an opening 124. The needle tip 122 is tapered so as to allow the needle 120 to puncture the body tissues more easily. The opening 124 may be located on a lateral side of the needle 120, thereby forming a side opening according to the invention. In the illustrated example, the opening 124 is located on the side of the needle 120 facing the other needle. In other terms, the openings 124 of the two needles 120 face each other.

The longitudinal lumen 121 and the suture thread end 22 are dimensioned so that the suture thread end 22 can be inserted into the longitudinal lumen 121 and move (i.e. slide) along the longitudinal lumen 121 up to the opening 124. The device 100 comprises a a projection mechanism for moving the suture thread end 22 inside and along the longitudinal lumen 121 and for projecting the suture thread end 22 out of the needle, through the opening 124. Different kinds of projection mechanisms 130 could be used. For instance, in the illustrated example, the projection mechanisms 130 may comprise a rod 125 sliding inside the longitudinal lumen 121 for pushing the suture thread end 22. The rod 125 may be moved by various appropriate mechanisms such as spring mechanisms, electric mechanisms, hydraulic mechanisms, etc. The projection mechanism 130 may be controlled by a controller such as a lever, a push-button, a joystick, etc. operable by a practitioner for projecting the suture thread ends 22 out of the needles. The controller may also control the needle movement from a retracted position to an extended position, or inversely.

As illustrated in FIG 1, the surgical suture device 100 may also comprise an anvil 110 distally spaced from the needle tips 122, i.e. in front of the needle tips 122 and spaced from them. The anvil 110 comprises a base 112 in front of the needle tips 122. In the example, the anvil 110 is fixed to the housing 140 of the device enclosing the projection mechanism 130 or to another part of the device 100. The space between the needle tips 122 and the base 112 may be adjustable. The space between the anvil 110 and the needle tips 122 is adjustable in order to adjust to the thickness of the tissue portions 1 and 2. After the base of the anvil 112 is introduced below the bottom surface of the tissue portions, the mechanism is triggered and both needles 120 traverse the tissue until they come close to or into contact with the anvil 110, at which point the suture thread ends 22 are released.

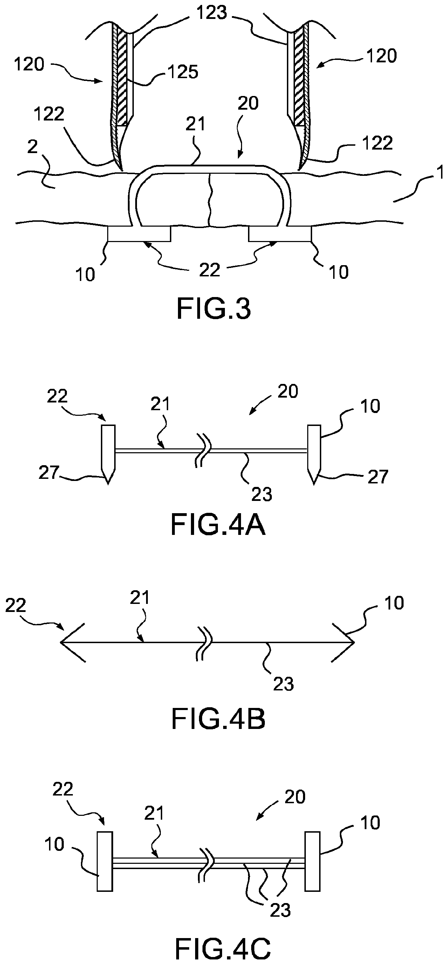

As illustrated in FIGS 4A to 4C, the suture thread 20 may have various structures and shapes. The suture thread 20 comprises two ends 22 with a first link 21 extending therebetween. The first link 21 may comprise one or more filaments 23, i.e. it may be monofilament or multifilament. When the link 21 is multifilament, the filaments 23 may be braided, twisted, etc. or separate from each other. In the examples of FIG 4A and 4B, the suture thread 20 comprises one single filament 23, whereas, in the example of FIG 2B, the suture thread 20 comprises three separate filaments 23 parallel with each other.

In the example, each needle 120 is movable through the body tissues 1, 2, from a retracted position where its tip 122 is away from the tissues to an extended position where its tip 122 extends beyond the tissues, after having punctured them. The needle movement direction is represented by a double arrow Y in FIG 1. In FIG 1, the needles 120 are shown in an intermediate position where the tips 122 are going through the tissue layer formed by the tissue portions 1, 2. In FIG 2, the needles 120 are shown in the extended position where the tips 122 are beyond the tissue layer. In FIG 3, the needles 120 are shown in a retracted position, after the suture thread has been discharged from them.

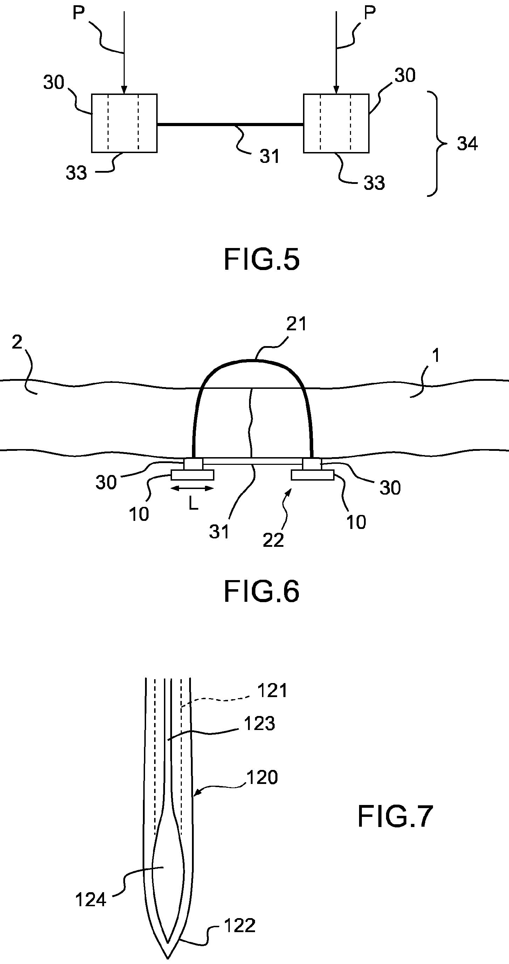

In the illustrated example, each hollow needle 120 comprises a longitudinal slot 123 extending along a lateral side of the needle 120. In particular, the longitudinal slot 123 is located on the side facing the other needle 120. In other terms, the longitudinal slots 123 of the two needles 120 face each other. The longitudinal slot 123 communicates with the longitudinal lumen 121 and extends longitudinally up to the opening 124 - see, in particular, FIG 7. As a result, when the two suture thread ends 22 slide, respectively, inside the two longitudinal lumens 121 of the needles 120, the link 21 moves along the longitudinal slots 123 of the needles 120.

Each suture thread end 22 forms an anchoring head 10 adapted for engagement with the tissues to be sutured. The anchoring heads 10 may have various shapes. In an example, the suture thread end 22 may have one or more barbs forming the anchoring head 10, i.e. one or more sharp projections extending backward and preventing extraction of the anchoring head 10 from the body tissues. In particular, as shown in FIG 4B, the suture thread end 22 may be arrow shaped, the barbs of the arrow point forming the anchoring head 10.

In another example, illustrated on FIGS 1, 2, 3, 4A and 6, the suture thread end 22 may have a T-shape with the head of the T (i.e. the upper horizontal bar of the T) forming the anchoring head 10, the leg of the T (i.e. the vertical bar of the T) being formed by the first link 21. In this example, when the T-head 10 slides inside the longitudinal lumen 121, it is oriented parallel to the longitudinal lumen 121 and substantially orthogonal to the tissue layer. However, once the T-head 10 is projected outside of the needle, its orientation changes and becomes inclined relative to the longitudinal lumen 121 and rather parallel to the tissue layer. Such a transverse orientation of the anchoring head 10 allows the anchoring head 10 to engage with the body tissues and prevents extraction of the anchoring head 10 from the body tissues, as illustrated in FIG 3.

Also, one end 27 of the upper bar (or head) of the T forming the anchoring head 10 may be pointed, as shown in FIG 4A. This allows the anchoring head 10 to better penetrate into the tissues, thereby providing a deeper penetration and/or a better engagement of the anchoring head 10 with the tissues.

In certain examples, several suture threads 20 may be loaded automatically and successively in the needles 120 so that several sutures can be made successively. Various kinds of loading mechanisms could be used for this purpose, including, for instance a loading mechanism with a spring loaded piston or an hydraulic piston pushing a stack of suture threads 20 towards proximal or loading openings of the needles 120.

According to another example illustrated in FIGS 5 and 6, the surgical suture device 100 comprises a lock-in unit 34 including first and second lock-in holders 30 connected together by a link 31. Each lock-in holder 30 cooperates with the anchoring head 10 of the suture thread 20 for preventing backward movement of the anchoring head 10 once the anchoring head 10 has passed through the body tissues. In other terms, after its projection, the anchoring head 10 engages with a lock-in holder 30 located in front of the needle tip 122. In other words, the lock-in holder 30 interacts with the suture thread ends 22 after they traverse through the tissue portions 1, 2. The lock-in holder 30 may be temporarily fixed on the anvil 110 (not shown). Also, several lock-in holders 30 may be positioned at the base of the anvil 110 where they can slide and remain in succession while being pushed, e.g., by a spring-loaded piston (not shown), and abutting against a stop (not shown) provided on the anvil 110. In other words, the lock-in holders 30 may be loaded automatically and successively on the anvil 110.

Each lock-in holder 30 has a through hole 33 extending in the movement direction Y of the needles, i.e. substantially orthogonal to the tissue layer. The through hole 33 has a cross section larger than the outer cross section of the needle 120 so that the needle (or at least the distal end portion thereof) can go through the hole 33, as illustrated by arrows P in FIG. 5. Such a lock-in holder 30 is intended to be used with a suture thread 20 having T-shaped anchoring heads 10 such as illustrated, for instance, in FIGS 1-3 and 4A. Once the anchoring head 10 has been discharged from the needle 120, its orientation changes and becomes transverse to the through hole 33 (and rather parallel to the tissue layer). With such a transverse orientation, the anchoring head 10 cannot go backward through the hole 33 because the through hole 33 has a cross section smaller than the length L of the anchoring head 10. As a result, the anchoring head is engaged with the lock-in holder 30, as illustrated in FIG 6, and both the lock-in unit 34 and the suture thread 20 participate to the stitch. Such cooperation between the lock-in unit 34 and the suture thread 20 allows, for example, a good apposition of the edges 1E, 2E of the tissue portions 1, 2, thereby avoiding invagination of the tissues.

While only some selected embodiments have been chosen to illustrate the present invention, it will be apparent to those skilled in the art from this disclosure that various changes and modifications can be made herein without departing from the scope of the invention as defined in the appended claims. For example, the size, shape, location or orientation of the various components can be changed as needed and/or desired. The structures and functions of one embodiment can be adopted in another embodiment. Further, it is not necessary for all advantages to be present in particular embodiments at the same time.

## Claims

1. A surgical suture device (100) for suturing body tissues comprising:
two hollow needles (120), each having a longitudinal lumen (121),
a suture thread (20) with two opposite ends (22) which are movable, respectively, inside and along the two longitudinal lumens (121),
a projection mechanism (130) for projecting the suture thread ends (22) out of the needles (120),
wherein each suture thread end (22) forms an anchoring head (10) for engagement with body tissues (1, 2) or a lock-in holder (30).

2. The surgical suture device of claim 1, wherein the suture thread end (22) has a T-shape, the anchoring head (10) being formed by the head of the T.

3. The surgical suture device of claim 1 or 2, comprising a lock-in holder (30) cooperating with the anchoring head (10).

4. The surgical suture device according to any of claims 1 to 3, wherein the anchoring heads (10) of the suture thread (20) are connected with each other by a first link (31).

5. The surgical suture device of claim 4, wherein each of the needles (120) comprises a longitudinal slot (123) extending along the longitudinal lumen (121) and communicating therewith, and wherein the first link passes through and is movable along the longitudinal slot (123).

6. The surgical suture device according to any of claims 1 to 5, comprising a lock-in unit with two lock-in holders (30) connected with each other by a second link (31).

7. The surgical suture device according to any of claims 1 to 6, wherein each of the needles has distal end portion with a side opening for discharging the suture thread end (22).

8. The surgical suture device according to any of claims 1 to 8, wherein each of the needles has a tip (122) and the device comprises an anvil (110) distally spaced from the needle tips (122).

9. The surgical suture device according to claim 9, wherein the anvil (110) holds the lock-in holders (30).
